(19) Europäisches Patentamt
European Patent Office
Office européen des brevets

(11) EP 1 000 029 B1

(12) EUROPEAN PATENT SPECIFICATION

(45) Date of publication and mention of the grant of the patent: **01.10.2003 Bulletin 2003/40**

(21) Application number: **98940159.1**

(22) Date of filing: **07.07.1998**

(51) Int Cl.⁷: **C07D 211/58**, A61K 31/445

(86) International application number: **PCT/EP98/04193**

(87) International publication number: **WO 99/002496 (21.01.1999 Gazette 1999/03)**

# (54) (+)-NORCISAPRIDE USEFUL FOR 5-HT3 AND 5-HT4 MEDIATED DISORDERS

(+)-NORCISAPRIDE VERWENDBAR BEI 5-HT3 UND 5-HT4 BEDINGTEN KRANKHEITEN

(+)-NORCISAPRIDE UTILES POUR TRAITER LES TROUBLES A MEDIATION 5-HT3 ET 5-HT4

(84) Designated Contracting States:
**AT BE CH CY DE DK ES FI FR GB GR IE IT LI LU NL PT SE**
Designated Extension States:
**AL LT LV MK RO SI**

(30) Priority: **11.07.1997 EP 97202161**
**04.03.1998 EP 98200661**

(43) Date of publication of application:
**17.05.2000 Bulletin 2000/20**

(73) Proprietor: **JANSSEN PHARMACEUTICA N.V.**
**2340 Beerse (BE)**

(72) Inventors:
- **HEYKANTS, Jozef, Jan, Pieter**
  **B-2340 Beerse (BE)**
- **MEGENS, Antonius, Adrianus, Hendrikus, Petrus**
  **B-2340 Beerse (BE)**
- **MEULDERMANS, Willem, Emiel, Gustaaf**
  **B-2340 Beerse (BE)**
- **SCHUURKES, Joannes, Adrianus, Jacobus**
  **B-2340 Beerse (BE)**

(56) References cited:
**EP-A- 0 076 530**
**EP-A- 0 299 566**
**EP-A- 0 640 601**
**EP-B- 0 423 133**
**WO-A-94/12494**
**WO-A-96/40133**
**WO-A-98/03173**
**FR-A- 2 717 174**

- **MEULDERMANS W ET AL: "EXCRETION AND BIOTRANSFORMATION OF CISAPRIDE IN DOGS AND HUMANS AFTER ORAL ADMINISTATION" DRUG METABOLISM AND DISPOSITION, vol. 16, no. 3, 1 May 1988, pages 403-409, XP000577694**
- **MEULDERMANS W ET AL: "EXCRETION AND BIOTRANFORMATION OF CISAPRIDE IN RATS AFTER ORAL ADMINISTRATION" DRUG METABOLISM AND DISPOSITION, vol. 16, no. 3, 1 May 1988, pages 410-419, XP000577692 cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention concerns (+)-norcisapride, and its pharmaceutically acceptable acid additions salts, a process for preparing said compound, and its use for the manufacture of a medicament for treating gastro-intestinal disorders while avoiding central nervous system effects.

Cisapride is a widely used gastrokinetic commercially known as "Prepulsid™". It is currently used primarily to treat gastro-esophagal reflux disease. This disease is characterized as the backward flow of the stomach contents into the esophagus. Other factors in the pathogenesis of the disease are delayed gastric emptying, insufficient esophageal clearing due to impaired peristalsis and the corrosive nature of the reflux material which can damage the esophageal mucosa.

Because of its activity as a prokinetic agent, cisapride may also be useful to treat dyspepsia, gastroparesis, constipation, postoperative ileus, and intestinal pseudo-obstruction.

Cisapride is metabolized mainly via the enzyme cytochrome P 450 3A4. As a result of extensive metabolization, unchanged cisapride accounts for less than 10% of urinary and fecal recovery following oral administration. The principal metabolite, found in plasma, feces and urine, described by Meuldermans W. et al. in *Drug Metab. Dispos. 16 (3) : 410-419, 1988*, is named "norcisapride" and is a racemic mixture of two enantiomers.

Racemic norcisapride is also disclosed in EP-A-0,076,530, published on 13 April 1983, as compound 211 having gastro-intestinal stimulating properties.

(+)-Norcisapride is one of the optical stereoisomers. The full chemical name is (+)-4-amino-5-chloro-*N*-(3-methoxy-4-piperidinyl)-2-methoxybenzamide, hereinafter referred to as "(+)-norcisapride". The term "(+)-norcisapride" and particularly the term "optically pure (+)-norcisapride" encompasses the (+)-stereoisomer which is substantially free of its (-)-stereoisomer.

WO 96/40133 teaches the use of (-)-norcisapride for treating digestive tract disorders, in particular for treating gastro-oesophageal reflux disease while substantially reducing adverse effects associated with the administration of racemic cisapride. The said document goes on to mention that optically pure (-)-norcisapride would be an effective antiemetic agent, useful as an adjunctive therapy in cancer treatment to alleviate nausea and vomiting induced by chemo- or radiotherapeutics.

WO-94/12494 discloses the use of known dimethylbenzofurans and dimethylbenzopyrans as $5HT_3$-antagonists. FR-2,717,174 discloses piperidinoalkyl 4-amino-5-chloro-2-methoxybenzoates as 5-HT4 agonists. WO-98/03173, published on 29 January 1998, discloses (+)-norcisapride and its use for the treatment of disorders of the central nervous sytem, as well as its use as an antiemetic agent.

It has now been found that (+)-norcisapride, in particular "optically pure (+)-norcisapride", also referred to as "(+)-norcisapride substantially free of its (-)-stereoisomer", has both $5\text{-}HT_3$ antagonistic and $5\text{-}HT_4$ agonistic properties and is further substantially devoid of central nervous system effects.

The present invention concerns (+)-norcisapride and in particular (+)-norcisapride substantially free of its (-)-stereo-isomer, the pharmaceutically acceptable acid addition salts thereof, and the use thereof for treating $5\text{-}HT_3$- and/or $5\text{-}HT_4$-mediated disorders while avoiding central nervous system effects. (+)-Norcisapride can be represented by the following chemical structure:

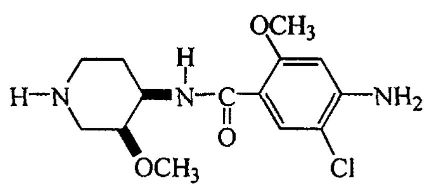 (I)

(+)-cis

In the above representation of the chemical structure of (+)-norcisapride, the bonds linking the NH-CO and the OCH groups to the piperidine ring are represented in bold to indicate that these two groups are in a cis configuration.

The term "(+)-norcisapride" and particularly the term "optically pure (+)-norcisapride" encompasses the (+)-stereoisomer which is substantially free of its (-)-stereoisomer. In particular, the term "substantially free of the (-)-stereoisomer" refers to a stereochemical mixture of norcisapride containing at least 90 % by weight of (+)-norcisapride and 10 % by weight or less of (-)-norcisapride. In a more preferred embodiment the term "substantially free of the (-)-stereoisomer" means that the norcisapride stereochemical mixture contains at least 98 % by weight of (+)-norcisapride, and 2 % or less of (-)-norcisapride. In a most preferred embodiment, the term "substantially free of the (-)-stereoisomer"

as used herein means that the composition contains greater than 99 % by weight of (+)-norcisapride. These percentages are based upon the total amount of norcisapride in the stereochemical mixture.

Compounds that are able to rotate the plane of polarized light are said to be optically active. In describing an optically active compound, the prefixes D and L or R and S are used to denote the absolute configuration of the molecule about its chiral center(s). The prefixes *d* and *l* or (+) and (-) are employed to designate the sign of rotation of plane-polarized light by the compound, with (-) and *l* meaning that the compound is levorotatory. A compound prefixed with (+) or *d* is dextrorotatory. For a given chemical structure, these compounds, called stereoisomers, are identical except that they are mirror images of one another. A specific stereoisomer may also be referred to as an enantiomer, and a mixture of such stereoisomers is often called a racemic mixture.

The amount of rotation $\alpha$ is not a constant for a given enantiomer, it depends on the length of the sample vessel, the temperature, the solvent and the concentration (for solutions) and the wavelength of light. Therefore, the specific rotation [$\alpha$] is defined so as to have a parameter which is independent of the length of the vessel and the concentration of the solution. The specific rotation $[\alpha] = \frac{\alpha}{lc}$, wherein $\alpha$ is the observed rotation, 1 is the length of the vessel in decimeters and c is the concentration in grams per milliliter. The specific rotation is usually given along with the temperature and wavelength, in this manner $[\alpha]_D^{20}$, wherein 20 is the temperature in degrees Celsius and D means that the rotation was measured with sodium D light, *i.e.* having a wavelength of 589 nm.

X-ray analysis of the L-tartaric acid salt of (+)-norcisapride, *i.e.* (+)-(3S,4R)-cis-4-amino-5-chloro-2-methoxy-*N*-(3-methoxy-4-piperidinyl)benzamide [R-(R*,R*)]-2,3-dihydroxybutanediotate monohydrate, unveiled the absolute configuration of (+)-norcisapride as (3S,4R). Hence, (+)-norcisapride has the following absolute stereochemistry :

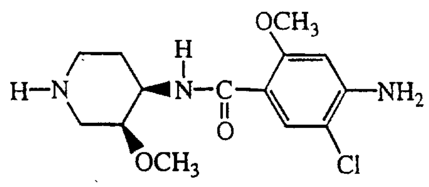

(3S,4R)-*cis*-4-amino-5-chloro-2-methoxy-*N*-(3-methoxy-4-piperidinyl)benzamide.

The pharmaceutically acceptable acid addition salts as mentioned hereinabove are meant to comprise the therapeutically active non-toxic acid addition salt forms which the (+)-norcisapride base is able to form. (+)-Norcisapride base can be converted in its pharmaceutically acceptable acid addition salts by treating (+)-norcisapride base with an appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic (*i.e.* butanedioic acid), maleic, fumaric, malic, tartaric, citric, methanesulfonic, ethanesulfonic, benzenesulfonic, *p*-toluenesulfonic, cyclamic, salicylic, *p*-aminosalicylic, pamoic and the like acids.

Conversely said acid addition salt forms can be converted by treatment with an appropriate base into the free (+)-norcisapride base form.

The term addition salt as used hereinabove also comprises the solvates which (+)-norcisapride as well as the salts thereof, are able to form. Such solvates are for example hydrates, alcoholates and the like.

Whenever used hereinafter, the term (+)-norcisapride is meant to also include its pharmaceutically acceptable acid addition salts, and its solvate forms.

(+)-Norcisapride base can be prepared as outlined in scheme 1.

Scheme I

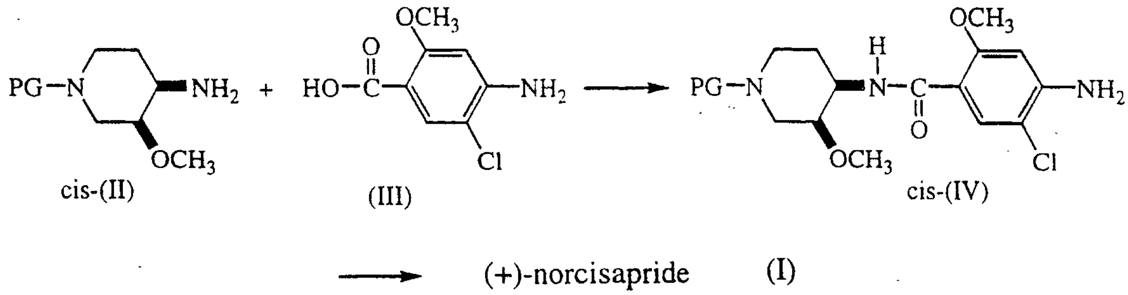

cis-(II) (III) cis-(IV)

(+)-norcisapride (I)

The amino and methoxy substituents on the piperidine ring in the intermediates of formula cis-(II) and reaction product cis-(IV) have the cis-configuration, *i.e.* the methoxy and the amino group are on the same side of the mean plane determined by the piperidine ring and is a racemic mixture of 2 enantiomers. To indicate this configuration, the bonds linking these functional groups are represented in bold, as in formula I. According to the reaction of Scheme 1, an intermediate of formula cis-(II), wherein PG is an appropriate protecting group such as, e.g. methyloxycarbonyl, ethyloxycarbonyl, *tert*-butyloxy-carbonyl, phenylmethyl and the like, is reacted with a carboxylic acid of formula (III), or a reactive functional derivative thereof, in an amide-forming reaction, thereby yielding intermediates of formula cis-(IV). The intermediates cis-(II) and (III) are reacted in the presence of a dehydrating agent such as dicyclohexylcarbodimide or cis-(II) is reacted with a reactive derivative of the carboxylic acid (III), e.g. an acid chloride or mixed anhydride. The reaction can be performed in a reaction-inert solvent such as, for example, dichloromethane or chloroform, and optionally in the presence of a suitable base such as, for example, sodium carbonate, potassium carbonate or triethylamine. Stirring may enhance the rate of the reaction. The reaction may conveniently be carried out at a temperature ranging between room temperature and the reflux temperature of the reaction mixture. Subsequently, the intermediates of formula cis-(IV) are separated into their enantiomers and each isolated enantiomer of intermediate cis-(IV) is converted into its corresponding norcisapride enantiomer by removing the protective group PG, depending on the nature of the goup PG, by acidic or basic solvolysis or by catalytic hydrogenation, e.g. by treatment with an inorganic base such as potassium hydroxide in an aqueous medium. Alternatively, the protective group PG of intermediates of formula cis-(IV) may be removed, thereby yielding the racemic mixture norcisapride, which is subsequently separated into its enantiomers.

The intermediates of formula cis-(IV) as prepared in scheme 1 are a mixture of enantiomers which can be separated from one another following art-known resolution procedures. The mixture of enantiomeric forms of the intermediates of formula cis-(IV) may be separated by conversion into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the intermediates of formula cis-(IV) involves liquid chromatography using a suitable chiral stationary phase. Suitable chiral stationary phases are, for example, polysaccharides, in particular cellulose or amylose derivatives. Commercially available polysaccharide based chiral stationary phases are ChiralCel™ CA, OA, OB, OC, OD, OF, OG, OJ and OK, and Chiralpak™ AD, AS, OP(+) and OT(+). Appropriate eluents or mobile phases for use in combination with said polysaccharide chiral stationary phases are hexane and the like, modified with an alcohol such as ethanol, isopropanol and the like.

Enantiomerically pure forms of intermediates of formula cis-(IV) may also be derived from the corresponding enantiomerically pure forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The present invention also provides for novel intermediates of formula (V). Said intermediates of formula (V) are intermediates of formula cis-(IV) wherein the piperidine ring has the absolute configuration (3S,4R) and PG is an appropriate protecting group such as methyloxycarbonyl, ethyloxycarbonyl, *tert*-butyloxycarbonyl, phenylmethyl and the like.

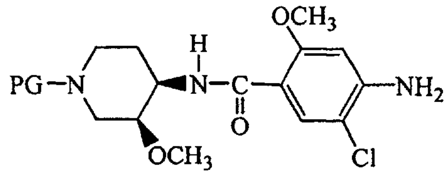

(V)

The starting materials and some of the intermediates are known compounds and are commercially available or may be prepared according to conventional reaction procedures generally known in the art. For example, an intermediate of formula cis-(II), *i.e.* cis-ethyl 4-amino-3-methoxy-1-piperidinecarboxylate, is described in EP-0,076,530 as intermediate 54, and an intermediate of formula cis-(IV), *i.e.* cis-ethyl 4-(4-amino-5-chloro-2-methoxybenzoylamino)-3-methoxy-1-piperidinecarboxylate is described in EP-0,076,530 as compound 168. Furthermore, intermediate (III), *i. e.* 4-amino-5-chloro-2-methoxybenzoic acid, is commercially available.

In view of its 5-$HT_3$-antagonising activity (+)-norcisapride is useful in the treatment of disorders associated with overstimulation of 5-$HT_3$-receptor activity. 5-$HT_3$-mediated disorders are, for example, emesis, e.g. cytotoxic drug and radiation induced emesis (Drugs 42(4), 551-568 (1991)), irritable bowel syndrome, especially diarrhea-predominant irritable bowel syndrome, and related disorders.

Or alternatively, (+)-norcisapride, and its pharmaceutically acceptable acid addition salts, are useful for the manufacture of a medicament for treating disorders or conditions associated with overstimulation of 5-$HT_3$-receptor activity or in general 5-$HT_3$ mediated diseases, *i.e.* emesis, cytotoxic drug and radiation induced emesis, irritable bowel syndrome and diarrhea-predominant irritable bowel syndrome.

In particular, (+)-norcisapride is an effective antiemetic agent, useful as an adjunctive therapy in cancer treatment to alleviate nausea and vomiting induced by chemo- or radio-therapeutics.

(+)-Norcisapride also has 5-$HT_4$-agonistic properties

Gastroparesis can be brought about by an abnormality in the stomach or as a complication of diseases such as diabetes, progressive systemic sclerosis, anorexia nervosa, after vagotomy or partial gastrectomy, and myotonic dystrophy. Dyspepsia is an impairment of the function of digestion, that can arise as a symptom of a primary gastrointestinal dysfunction, especially a gastrointestinal dysfunction related to an increased muscle tone or as a complication due to other disorders such as appendicitis, galbladder disturbances, or malnutrition. (+)-Norcisapride can thus be used either to take away the actual cause of the condition or to relief the patients from symptoms of the conditions. The symptoms of dyspepsia may also arise due to the intake of chemical substances, e.g. Selective Seretonine Reuptake Inhibitors (SSRI's), such as fluoxetine, paroxetine fluvoxamine, sertraline and the like. Other symptoms that can be treated comprise post-operative ileus, which is an obstruction or a kinetic impairrment in the intestine due to a disruption in muscle tone following surgery; symptoms of X-ray or endoscopy negative upper digestive discomfort; in infants: chronic and excessive regurgitation or vomiting; intestinal pseudo-obstruction, associated with motility dysfunctions resulting in insufficient propulsive peristaltism and in stasis of gastric and intestinal contents; constipation, which can result from conditions such as lack of intestinal muscle tone or intestinal spasticy; in particular restoration of colonic propulsive motility as a long-term treatment of chronic constipation.

Hence, the use of (+)-norcisapride for the manufacture of a medicine for treating disorders or conditions involving understimulation of the 5-$HT_4$-receptor activity is provided. Also the use of (+)-norcisapride for the manufacture of a medicament for treating 5-$HT_4$-mediated disorders, such as, e.g. gastro-oesophageal reflux, dyspepsia or gastroparesis, is provided. Both prophylactic and therapeutic treatment are envisaged.

In an aspect, the use of (+)-norcisapride for the manufacture of a medicine for treating eating disorders such as, e.g. anorexia, is also provided. Hence, a method of treating eating disorders such as, e.g. anorexia, in warm-blooded animals which comprises administering to said warm-blooded animal a therapautically effective amount of (+)-norcisapride, is provided.

Consequently, the present invention also provides a method of treating gastro-intestinal disorders in a warm-blooded animal which are simultaneously associated with an understimulation of the 5-$HT_4$-receptor activity and an overstimulation of the 5-$HT_3$-receptor activity which comprises administering to said warm-blooded animal a therapeutically effective amount of (+)-norcisapride. Hence, the use of (+)-norcisapride for the manufacture of a medicine for treating gastro-intestinal disorders simultaneously associated with an understimulation of the 5-$HT_4$-receptor activity and an overstimulation of the 5-$HT_3$-receptor activity.

Additionally, (+)-norcisapride has a synergistic effect with osmotic agents to obtain intestinal lavage, *i.e.* an induced form of diarrhea. Hence, the present invention is also concerned with the use of (+)-norcisapride for the manufacture of a medicament that will improve the intestinal cleansing by a laxative, in particular an osmotic agent. Consequently, a method of treatment is claimed whereby an effective amount of (+)-norcisapride is administered in combination with a laxative. Further, a method is provided to accelerate and/or enforce the action of laxatives, especially osmotic agents.

The patients envisaged in this treatment are people whose bowel needs to be cleaned prior to diagnostic or surgical procedures. Another group of patients are those patients who are to be prevented from straining at the stool, these patients include people suffering from hernia or cardiovascular disease. In addition, the combination of the present invention can be indicated, both before and after surgery, to maintain soft feces in patients with hemorrhoids and other anorectal disorders.

Osmotic agents at cathartic doses are frequently employed prior to radiological examination of the gastrointestinal tract, kidneys, or other abdominal or retroperitoneal structures and prior to elective bowel surgery. Hence, also for these applications the combination of (+)-norcisapride and a laxative may be useful.

Furthermore the combination of (+)-norcisapride and a laxative can also be used in the treatment of drug overdosage and poisoning, by removing agents from the intestine. Saide combination may also be employed in further combination with certain anthelmintics.

Laxatives are drugs that promote defecation. Precise mechanisms of action of many laxatives remain uncertain because of the complex factors that affect colonic function, prominent variations of water and electrolyte transport among experimental species and preparations, and a certain costiveness of research in this area. Three general mechanisms of laxative action can be described. (1) By their hydrophilic or osmotic properties, laxatives may cause retention of fluid in colonic contents, thereby increasing bulk and softness and facilitating transit. (2) Laxatives may act, both directly and indirectly, on the colonic mucosa to decrease net absorption of water and NaCl. (3) Laxatives may increase intestinal motility, causing decreased absorption of salt and water secondary to decreased transit time. Mostly one recognizes three major classes of laxatives, *i.e.* 1) dietary fiber and bulk-forming laxatives, 2) saline and osmotic laxatives and 3) stimulant laxatives. (see *Goodman and Gilman, seventh edition, pp 994 to 1003*).

Saline and osmotic laxatives are the primary class of laxatives envisaged in this invention. Saline and osmotic laxatives include various magnesium salts; the sulfate, phosphate, and tartrate salts of sodium and potassium; the dissacharide lactulose; glycerin; and sorbitol. They are poorly and slowly absorbed and act by their osmotic properties in the luminal fluid. Two examples of these osmotic agents which are commercially available for intestinal cleansing are KleanPrep® and GoLytely®. The KleanPrep® solution consists of polyethyleneglycol 3350 (59 g/l), sodium sulfate (5.685 g/l), sodium hydrogen-carbonate (1.685 g/l), sodium chloride (1.465 g/l), potassium chloride (0.7425 g/l), asparatate (0.0494 g/l) and vanille (0.3291 g/l).

Furthermore, (+)-norcisapride, and its pharmaceutically acceptable salts, are substantially devoid of central nervous system effects, in particular the absence of central dopamine antagonistic and central serotonin antagonistic activity.

It has been found that (+)-norcisapride is metabolized through a pathway other than that which is responsible for the metabolism of cisapride. The latter apparently is metabolized mainly through the cytochrome p450 system. Certain therapeutic agents that inhibit the main metabolic pathway of cisapride can give rise to undesirably high cisapride blood levels that may cause side effects. Co-medication of such therapeutic agents with cisapride may be dissuasive, which will not be the case with (+)-norcisapride.

The present invention also relates to oral dosage forms comprising the (L)-tartrate or (D)-tartrate salt forms of (+)-norcisapride. Said salt forms advantageously show a dissolution profile that is less pH dependent. The absorption and bioavailability of drugs having a pH dependent solubility profile, when taken orally, can be affected by the presence of food in the gastro-intestinal (GI) tract, in particular in the stomach. The gastric residence time of a drug is usually significantly longer in the presence of food than in the fasted state, i.e. the presence of food will prolong the stay of the drug in the relatively acidic medium of the stomach. If the bioavailability of a drug is affected beyond a certain point due to the presence of food in the GI tract, the drug is said to exhibit a "food effect" or show a drug-food interaction. In order to have adequate bioavailability, such drugs often have to be administered under specific feeding conditions, e.g. before the intake of a meal, as is the case with cisapride. Oral dosage forms comprising said tartrate salts have the advantage that the bioavailability of (+)-norcisapride is independent of the intake of a meal. Such dosage forms therefore are attractive because patients do not always have the necessary discipline to take their medication at the optimum point in time resulting in variable and sometimes inadequate efficacy. This in particular is the case in pediatric applications. Such dosage forms show the additional advantage of "*pro re nata*" administration, i.e. a symptom driven administration. Furthermore, because of their pH independence, said oral dosage forms can be co-administered with agents that alter the pH of the stomach, for example agents that increase the pH of the stomach. Examples of such co-medication are antacids, such as aluminum containing antacids, e.g. $Al(OH)_3$, calcium containing antacids, e.g. $CaCO_3$, or magnesium containing antacids, e.g. $Mg(OH)_2$; $H_2$-antagonists, e.g. cimetidine, ranitidine, famotidine, nizatidine, roxatidine and the like; or proton pump inhibitors, e.g. omeprazole, lansoprazole, rabeprazole, pantoprazole and the like. Hence there are provided dosage forms, preferably solid dosage forms for oral administration, comprising

(+)-norcisapride tartrate and any of these antacids, as a combined preparation for simultaneous, separate or sequential use.

Therefore in a further aspect, the present invention concerns pharmaceutical formulations, in particular solid dosage forms, preferably for oral administration, suitable for rapid dissolution comprising as active ingredient (+)-norcisapride L- or D-tartrate and a suitable carrier. The term "suitable for rapid dissolution" refers to the fact that active ingredient can dissolve from the dosage form for more than 60 % within 1 hour in a pH range from 1 to 7. Dissolution can be measured according to standard methods described in the European Pharmacoeipea or as set forth in USP test <711> in a USP-2 dissolution apparatus (described in US Pharmacopeia XXII, pp 1578-1579). In order to have rapid dissolution the excepients should be selected such that the tablets disintegrates sufficiently fast, in particular in less than about 30 minutes, or less than 20 minutes and more particularly in less than about 15 minutes, preferably less than about 3, or 1.5 minutes. In still a further aspect, the invention concerns a method of treating patients suffering from gastrointestinal disorders, without drug-food interaction, comprising the administration of (+)-norcisapride (D)- or (L)-tartrate and of treating gastrointestinal disorders in patients taking medication that increases the pH of the stomach; or the use for the manufacture of a dosage form without drugfood interaction for the treatment of said disorders.

The formulations of the present invention may optionally include an anti-flatulent, such as simethicone, alpha-D-galactosidase and the like.

To prepare the pharmaceutical compositions of this invention, an effective amount of the particular compound, in base or acid addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on, as an ointment. Acid addition salts of (I) due to their increased water solubility over the corresponding base form, are obviously more suitable in the preparation of aqueous compositions.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

For oral administration, the pharmaceutical compositions may take the form of solid dose forms, for example, tablets (both swallowable-only and chewable forms), capsules or gelcaps, prepared by conventional means with pharmaceutically acceptable excipients such as binding agents, fillers or diluents, lubricants, disintegrants, wetting agents (e.g. sodium lauryl sulphate), and other excipients such as coloring agents and pigments.

The tablets may be coated by methods known in the art.

Binding agents may be acacia, alginic acid, carboxymethylcellulose (sodium), cellulose (microcrystalline), dextrin, ethylcellulose, gelatin, glucose (liquid), guar gum, hydroxypropyl cellulose, hydroxypropyl methylcellulose, methylcellulose, polyethylene oxide, polyvinylpyrrolidone (povidone), or starch (pregelatinized), hydroxypropyl methylcelluloses, especially the low-viscosity hydroxypropyl methylcelluloses.

Examples of fillers or diluents are spray-dried or anhydrous lactose, sucrose, dextrose, mannitol, sorbitol, starch, cellulose (e.g. microcrystalline cellulose; Avicel™), hydrated or anhydrous dibasic calcium phosphate, and others known in the art, or mixtures thereof. For instance, a mixture of lactose and micro crystalline cellulose can be used. Lactose is used as a pure diluent, while microcrystalline cellulose is a filler that has the property of yielding tablets with an appropriate hardness and it has disintegrant properties because cellulose fibers swell in contact with water. A preferred form of lactose is lactose monohydrate DC, in particular spray-dried lactose monohydrate (Pharmatose DCL

11™). Fillers or diluents may be present in a range from about 50 % to about 95 %, or from about 65 % to about 90 %, or from about 66 % (w/w) to about 86 %, in particular about 75%(all w/w) based on the total weight of the tablet or tablet core. The 75% mixture is commercially available under the tradename MICROCELAC® which is preferably present in an amount ranging from about 80 % (w/w) to 95 % (w/w) based on the total weight of the tablet or the tablet core in the case of film coated tablets.

Examples of lubricants are magnesium stearate, talc, silica, stearic acid, sodium stearyl fumarate, magnesium lauryl sulfate, hydrogenated vegetable oil and others known in the art. Lubricants generally are present in an amount ranging from about 0.2 % (w/w) to 7.0 % (w/w) based on the total weight of the tablet or the tablet core in the case of film-coated tablets. Interestingly, lubricants are present in amounts ranging from about 0.5 % (w/w) to about 3.0 % (w/w). Preferably, lubricants are present in amounts ranging from about 0.9 % (w/w) to about 1.25 % (w/w).

Disintegrants comprise starch, e.g. maize starch, pregelatinised starch, sodium starch glycolate (Explotab®), crosslinked povidone, crosslinked sodium carboxymethylcellulose, clays, microcrystalline cellulose (Avicel®), alginates, gums, carmellose sodium, also referred to as croscarmellose sodium, and others known in the art. The disintegrant may be present in an amount of about 2 % (w/w) to about 15 % (w/w) or from about 3 % (w/w) to about 10 % (w/w).

As used herein, percentages are weight per weight (w/w) and represent the ratio (in percent) of the ingredient or the excipient based on the total weight of the tablet (or in the case of coated tablets of the tablet core).

Usually, tablet blends are dry-granulated or wet-granulated before tableting. In some instances, in particular when using the (D)- or (L)-tartrates it may be possible to prepare tablets using direct compression techniques, which may give a better dissolution profile.

Liquid preparations for oral administration may take the form of, for example, solutions, syrups or suspensions, or they may be presented as a dry product for constitution with water or other suitable vehicle before use. Such liquid preparations may be prepared by conventional means, optionally with pharmaceutically acceptable additives such as suspending agents (e.g. sorbitol syrup, methylcellulose, hydroxypropyl methylcellulose or hydrogenated edible fats); emulsifying agents (e.g. lecithin or acacia); non-aqueous vehicles (e.g. almond oil, oily esters or ethyl alcohol); and preservatives (e.g. methyl or propyl p-hydroxybenzoates or sorbic acid).

Pharmaceutically acceptable sweeteners comprise preferably at least one intense sweetener such as saccharin, sodium or calcium saccharin, aspartame, acesulfame potassium, sodium cyclamate, alitame, a dihydrochalcone sweetener, monellin, stevioside or sucralose (4,1',6'-trichloro-4,1',6'-trideoxy*galacto*sucrose), preferably saccharin, sodium or calcium saccharin, and optionally a bulk sweetener such as sorbitol, mannitol, fructose, sucrose; maltose, isomalt, glucose, hydrogenated glucose syrup, xylitol, caramel or honey.

Intense sweeteners are conveniently employed in low concentrations. For example, in the case of sodium saccharin, the concentration may range from 0.04% to 0.1% (w/v) based on the total volume of the final formulation, and preferably is about 0.06% in the low-dosage formulations and about 0.08% in the high-dosage ones. The bulk sweetener can effectively be used in larger quantities ranging from about 10% to about 35%, preferably from about 10% to 15% (w/v).

Optionally, flavors may be incorporated in the composition, for example to mask bitter taste. Flavors which can mask the bitter tasting ingredients in lower-dosage formulations are preferably fruit flavours such as cherry, raspberry, black currant or strawberry flavour. A combination of two flavours may yield very good results. In higher-dosage formulations stronger flavours may be required such as Caramel Chocolate flavour, Mint Cool flavour, Fantasy flavour and the like pharmaceutically acceptable strong flavours. Each flavour may be present in the final composition in a concentration ranging from 0.05% to 1% (w/v). Combinations of said strong flavours are advantageously used. Preferably a flavour is used that does not undergo any change or loss of taste and colour under the acidic conditions of the formulation.

The compounds of the invention may also be formulated as depot preparations. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example as a sparingly soluble salt.

The compounds of the invention may be formulated for parenteral administration by injection, conveniently intravenous, intramuscular or subcutaneous injection, for example by bolus injection or continuous intravenous infusion. Formulations for injection may be presented in unit dosage form e.g. in ampoules or in multidose containers, with an added preservative. The compositions may take such forms as suspensions, solutions or emulsions in oily or aqueous vehicles, and may contain formulatory agents such as isotonizing, suspending, stabilising and/or dispersing agents. Alternatively, the active ingredient may be in powder form for constitution with a suitable vehicle, e.g. sterile pyrogen-free water before use.

The compounds of the invention may also be formulated in rectal compositions such as suppositories or retention enemas, e.g. containing conventional suppository bases such as cocoa butter or other glycerides.

For intranasal administration the compounds of the invention may be used, for example, as a liquid spray, as

a powder or in the form of drops.

In general it is contemplated that a therapeutically effective amount would be from about 0.001 mg/kg to about 2 mg/kg body weight, preferably from about 0.02 mg/kg to about 0.5 mg/kg body weight. Suitable solid oral dosage form when in a unit dose form comprises the equivalent of about 0.1 mg to 100 mg of the active ingredient, more particularly are of about 1 mg to about 50 mg to about 20 mg A method of treatment may also include administering the active ingredient on a regimen of sevaral intakes per day, e.g. 2-4 intakes per day.

Experimental part

Hereinafter "ACN" means acetonitrile, "$CH_3OH$" means methanol and "DIPE" means diisopropylether.

Of some compounds of the present invention the absolute stereochemical configuration was not experimentally determined. In those cases the stereochemically isomeric form which was first isolated is designated as "B" and the second as "A", without further reference to the actual absolute stereochemical configuration.

The optical rotation (OR), or specific rotation $[\alpha]^{20,D}$, measurements were done with a Perkin Elmer model 241. The rotatation $[\alpha]$ was measured at a temperature of 20°C, using the sodium D line (wavelength is 589 nm) and a cuvette with a path length of 100 mm. The compound was dissolved in methanol with a concentration of 1% w/v. Chiralpak AD (amylose 3,5 dimethylphenyl carbamate) is a chiral stationary phase column material purchased from Daicel Chemical Industries, LTd, in Japan.

A. Synthesis of (+)-norcisapride

Example A.1

Cis-ethyl 4-(4-amino-5-chloro-2-methoxybenzoylamino)-3-methoxy-1-piperidine carboxylate (0.12 mol, 46 g) (exemplified in EP-0,076,303 as compound No. 168) was separated into its enantiomers by chiral column chromatography over Chiralpak AD (2 kg; internal diameter column (ID): 110 mm, eluent: n-hexane/ethanol 70/30; injection: 5 g/litre, flow-rate : 400 ml/minute, detection : UV at 254 nm). Two desired fraction groups were collected and their solvent was evaporated. The residue of the first eluting enantiomer was dissolved in methanol, filtered over dicalite, and the solvent was evaporated. The residue was suspended in DIPE, then cooled to 0°C, and the resulting precipitate was filtered off and dried (vacuum, 40°C), yielding 18 g (-)-cis-ethyl 4-(4-amino-5-chloro-2-methoxybenzoylamino)-3-methoxy-1-piperidine-carboxylate (enantiomer 1; OR (-); mp: 164°C); $[\alpha]_D^{20}$ = -55.88° (c = 1 % w/v in $CH_3OH$) (interm. 1). Said intermediate 1 had an optical purity better than 99%. The residue of the second eluting enantiomer was dissolved in methanol, filtered over dicalite, and the solvent was evaporated. The residue was suspended in DIPE, then cooled to 0°C, and the resulting precipitate was filtered off and dried (vacuum, 40 °C), yielding 17.6 g (+)-cis-ethyl 4-(4-amino-5-chloro-2-methoxybenzoylamino)-3-methoxy-1-piperidinecarboxylate (enantiomer 2; OR (+); mp: 164°C); $[\alpha]_D^{20}$ = +55.10° (c = 1 % w/v in $CH_3OH$) (interm. 2), having an optical purity higher than 98%.

Example A.2

A mixture of intermediate (2) (0.042 mol, 16 g) and potassium hydroxide (23 g) in 2-propanol (230 ml) was stirred and refluxed for 6 hours. The reaction mixture was cooled and the solvent was evaporated. Water was added. The solvent was evaporated. The residue was suspended in water, then filtered and the solid was dissolved in dichloromethane. The organic solution was washed with water, dried, filtered and the solvent was evaporated. The residue was crystallized from ACN. The precipitate was filtered off and dried (vacuum), yielding fraction 1 (57%; mp: 188°C). This fraction was recrystallized from ACN. The precipitate was filtered off and dried, yielding fraction 2 (5.3 g). All crystallization filtrates were collected and the solvent was evaporated. The residue, as well as fraction 2 were purified by column chromatography over silica gel (eluent: $CH_2Cl_2/(CH_3OH/NH_3)$ 90/10). The pure fractions were collected and the solvent was evaporated. The solid residues were dried, yielding 5.6 g of (+)-cis-4-amino-5-chloro-2-methoxy-*N*-(3-methoxy-4-piperidinyl)benzamide (*i.e.* (+)-norcisapride); $[\alpha]_D^{20}$ = +5.60° (c = 1 % w/v in $CH_3OH$) (compound 1).

Example A.3

A mixture of intermediate (1) (0.044 mol, 17 g) and potassium hydroxide (24 g) in 2-propanol (250 ml) was stirred and refluxed for 6 hours. The reaction mixture was cooled and the solvent was evaporated. Water was added. The solvent was evaporated. The residue was stirred in water, then filtered and the solid was crystallized from ACN. The precipitate was filtered off, and dissolved in DCM. The organic solution was washed with water, dried, filtered and the solvent was evaporated. The residue was crystallized from ACN. The precipitate was filtered off and dried, yielding 7 g (54%) of (-)-cis-4-amino-5-chloro-2-methoxy-*N*-(3-methoxy-4-piperidinyl)benzamide (i.e. (-)-norcisapride); $[\alpha]_D^{20}$ =

-6.09° (c = 50.90 mg in 5 ml in $CH_3OH$) (compound 2).

Example A.4

To a stirred solution of (+)-cis-4-amino-5-chloro-2-methoxy-*N*-(3-methoxy-4-piperidinyl)benzamide (compound 1) in a mixture of water and methanol was added a solution of [R(R*,R*)]-2,3-dihydroxybutanedioic acid (L-tartaric acid) in a mixture of water and methanol and the product was allowed to crystallize. It was filtered off and dried, yielding (+)-(3S,4R)-cis-4-amino-5-chloro-2-methoxy-*N*-(3-methoxy-4-piperidinyl)benzamide [R(R*,R*)]-2,3-dihydroxybutanedioate monohydrate. The X-ray diffraction measurements were run on a Siemens P4 four-cirle diffractometer.

B. Pharmacological examples

Example B. 1. $MgSO_4$ induced intestinal lavage

Beagle dogs (of both sexes and varying in body weight, 16 hours deprived from food) were orally pretreated with (+)-norcisapride or solvent and challenged 1 hour later with an oral administration of magnesium sulphate ($MgSO_4$. $7H_2O$, 64g/l, 0.26M; 200 ml). The onset of $MgSO_4$-induced intestinal lavage was assessed over a period of 4 hours after challenge. Liquid stools within 4 hours occured almost never in control animals treated with distilled water (2.5% false positives; n=200) and were considered to reflect significant acceleration of the $MgSO_4$-induced intestinal lavage. Doses in the active dose range were given to five animals each, tested in separate experimental sessions including solvent-treated control animals. All-or-none criteria, based on the distribution of the results obtained in a large number of solvent-treated animals, were used to calculate $ED_{50}$-values (effective dose) and 95% confidence limits according to Finney's iterative method (Finney, 1962).
As illustrated in table B.1, a small increase in dose from 0.032 mg/kg to 0.15 mg/kg was sufficient to obtain diarrhoea with 1 hour after challenge with magnesium sulphate.

Table B.1 :

| $ED_{50}$ values (95% confidence limit, mg/kg, p.o.) of (+)-norcisapride for promoting magnesium sulphate-induced intestinal lavage in dogs at 1 hour after p.o. administration. $ED_{50}$'s for obtaining liquid stools within 1 to 4 hours after challenge with magnesium sulphate are listed. | |
|---|---|
| Effect | $ED_{50}$ values (mg/kg, p.o.) |
| Onset of diarrhoea with 4 hours | 0.032 |
| Onset of diarrhoea with 3 hours | 0.049 |
| Onset of diarrhoea with 2 hours | 0.064 |
| Onset of diarrhoea with 1 hour | 0.15 |

Example B.2 : "apomorphine, tryptamine, norepinephrine (ATN) test in rats"

The absence of central dopamine antagonistic and serotonin antagonistic activity of the subject compounds is evidenced by the experimental data obtained in the combined apomorphine (APO), tryptamine (TRY) and norepinephrine (NOR) test in rats. Said combined apomorphine, tryptamine and norepinephrine test is described in Arch. Int. Pharmacodyn., 227, 238-253 (1977) and provides an empirical evaluation of the relative specificity with which drugs may effect particular neurotransmitter systems centrally (CNS) as well as peripherally. In this test, rats were observed for effects or responses indicating peripheral and central activity. Central dopamine antagonism is evaluated by challenging rats, subcutaneously pretreated with different doses of the test compound, with apomorphine which is a dopamine agonist. Next, serotonin antagonism is evaluated by challenging the same rats, subcutaneously pretreated with different doses of the test compound, with tryptamine which is an agonist at serotonin $5HT_2$-receptors. Both central and peripheral serotonin antagonism can be assessed in this test. Centrally acting serotonin antagonists are potential antipsychotic drugs, in particular when simultaneously displaying dopamine antagonism in the first part of this test. Finally, $\alpha$-adrenergic antagonistic activity of the test compounds is evaluated by challenging the same rats, subcutaneously pretreated with different doses of the test compound, with norepinephrine which is an $\alpha$-adrenergic agonist.

The experimental data are summarized in Table B.2 and expressed as $ED_{50}$ values in mg/kg body weight, which are defined as the dose at which each of the tested compounds protects 50% of the tested animals from a relevant response evoked by the above-mentioned challenging substances. Column APO lists the results of the apomorphine challenge, indicating central dopamine antagonistic activity. Column TRY convulsions and TRY hyperaemia

list the results of the tryptamine challenge, indicating central and peripheral serotonin antagonistic activity respectively. Column NOR lists the results of the norepinephrine challenge, indicating α-adrenergic agonist activity. The favourable pharmacological properties of (+)-norcisapride lie in the absence of central dopamine (column APO) and central serotonin (column TRY convulsions) antagonistic activity.

Table B.2 :

| Compound | Combined test in rats, $ED_{50}$ in mg/kg | | | |
|---|---|---|---|---|
| | APO | TRY convulsions | TRY hyperaemia | NOR |
| (+)-norcisapride | > 40 | > 40 | 4.7 | > 40 |

Example B.3 : "5-hydroxykynuramine antagonism on the guinea-pig ileum".

5-$HT_3$ receptors were shown to play an important role in vomiting. Since 5-OH-K (5-hydroxykynuramine) is a specific agonist at the 5-$HT_3$ receptor, this test evaluates the potential 5-$HT_3$ antagonistic effects.

Guinea pigs of both sexes, body weight ± 450 g, were killed by decapitation. Non-terminal, (distal 10 cm discarded), intact ileum segments, strips were cleansed, 4.5 cm long, were vertically suspended with a preload of 0.75 g in an 100 ml bath for isotonic recording; [(Displacement Transducer Control Unit; Janssen Scientific Instrument Division), HP7 DGDT-1000 (Hewlett-Packard CY)]. The organ bath was filled with a Tyrode solution, (37.5°C), gassed with a mixture of 95% $O_2$ and 5% $CO_2$. After a stabilisation period of 20 minutes, methacholine ($3.10^{-6}$ M, 30 seconds contacttime) was added to the bathfluid in order to evaluate the potential maximal contraction, whereafter the bathfluid was refreshed. Subsequently, the serotonin agonist 5-OH-K (30 seconds contact time) was added (final concentration $3.10^{-6}$ M) at intervals of 10 minutes, the bath fluid being refreshed 30 seconds after each addition. After reaching reproducible contractile effects, a single dose of the test compound was added to the bath fluid followed 5 minutes later by addition of 5-OH-K. After establishing the log concentration-responds curve of the test compound, $IC_{50}$ values reducing the 5-OH-K induced effect by 50% were calculated by linear regression analysis.

Compound 1, *i.e.* (+)-norcisapride has an $IC_{50}$ of 93 nM; and compound 2, *i.e.* (-)-norcisapride has an $IC_{50}$ of 450 nM.

Example B.4 : "Guinea-pig ileum coaxial stimulation"

Dunkin-Hartley guinea-pigs of either sex (weighing between 600-900 g) were killed by decapitation. The ileum was removed and cleansed with warmed and oxygenated Krebs-Henseleit solution. Parts of the ileum (15 cm) were slipped over a glass pipette. The longitudinal muscle layer with myenteric plexus was removed by means of a cotton thread moistened with Krebs solution. Strips with a length of 8 cm were folded and these strips (4 cm) were mounted between two platinum electrodes (8 cm length, 0.5 cm apart). The strips were suspended with a preload of 1.5 g in 100 ml Krebs-Henseleit solution (37.5°C), gassed with a mixture of 95 % $O_2$ and 5 % $CO_2$. The preparations were excited with single rectangular stimuli [1 ms; 0.1 Hz; submaximal response (current leading to 80 % of maximal response), from a programmable stimulator (Janssen Scientific Instruments Division)]. Contractions were measured isometrically (Statham UC2, Janssen Scientific Instruments amplifier, Kipp BD-9 pen-recorder). During the stabilization period of 30 minutes, the strips were repeatedly stretched, in order to obtain a steady state tension of 1.5 g. Before starting the electrical stimulation, a cumulative concentration response curve of acetylcholine ($3.10^{-9}$, $10^{-8}$, $3.10^{-8}$ and $10^{-7}$ M) was given. The bath fluid was replaced with fresh Krebs solution and the strips were allowed to stabilize for another 30 minutes. Subsequently, the strips were stimulated electrically (power stimulator) at a frequency of 0.1 Hz for 1 ms. The voltage was increased by steps of 2 V (maximum 15 V) until maximum force development was observed. The twitch response was decreased (by voltage reduction) to about 80 % of that operative at maximal voltage. By adjusting the voltage carefully it was possible to obtain a submaximal twitch response which did not vary over at least 2 hours. When the twitch responses were stable for at least 15 minutes, the test compound was added to the bath fluid for 30 minutes. If the test compound caused less than 50 % inhibition, cisapride $3.10^{-7}$ M was added to the bath fluid to find out whether the test compound could antagonize the stimulatory effect of cisapride. If the test compound caused more than 50 % inhibition, naloxone $10^{-7}$ M was added to find out whether the inhibition was mediated via opiate receptors. After the addition of either cisapride or naloxone, a supramaximal stimulation was given again. Afterwards, the electrical stimulation was discontinued and a second cumulative concentration-response curve with acetylcholine was given. These two cumulative concentration-response curves of acetylcholine were given in order to distinguish effects via a decreased acetylcholine release, from a direct anticholinergic effect or to distinguish effects via enhanced release of acetylcholine from sensitization of muscarinic receptors. The $EC_{50}$ *(i.e.* the concentration that stimulates the response to electrical stimulation by 50%) of a compound, was calculated using linear regression analysis when the

test compound causes stimulation.

Compound 1, *i.e.* (+)-norcisapride has an $EC_{50}$ of 0.6μM; and compound 2, *i.e.* (-)-norcisapride has an $EC_{50}$ of 5 μM.

Example B.5 : "Guinea-pig colon coaxial stimulation"

Guinea-pigs of both sexes (body weight ± 450 g), were killed by decapitation. The colon ascendens was removed and rinsed, whereafter segments of approximately 3 cm length were prepared. These strips were suspended in an organ bath (100 ml) and connected to an isotonic transducer under a preload of 2 g; [Displacement Transducer Control Unit (Janssen Scientific Instrument Division); HP 7 DGDT 1000 (Hewlett-Packard)]. The organ bath was filled with a DeJalon solution (37.5°C), gassed with a mixture of 95 % $O_2$ and 5 % $CO_2$. After a stabilization period of 20 minutes, methacholine $3.10^{-6}$ M was added to the bathfluid in order to evaluate the potential maximal effect of the strip (contact time 30 seconds); thereafter, the bathfluid was refreshed. This was repeated at intervals of 10 minutes until a reproducible answer was obtained after addition of methacholine. Thereafter, the test compound was added for 10 minutes. Afterwards, without refreshing the bathfluid, cisapride $3.10^{-7}$ M was added to evaluate a possible $5HT_4$ effect. When the contraction, caused by cisapride obtained a stable maximal level, methacholine $3.10^{-6}$ M to evaluate a potential anticholinergic effect, was added to the bath fluid until a maximal contraction was obtained. The $EC_{50}$ (*i.e.* the concentration that stimulates the maximal tone effect by 50%) of a compound, was calculated using linear regression analysis.

Compound 1, *i.e.* (+)-norcisapride has an $EC_{50}$ of 1.9 μM; and compound 2, *i.e.* (-)-norcisapride has an $EC_{50}$ of 11 μM.

C. Pharmaceutical composition examples

C.1 Film-coated tablet

The active ingredient, the lactose, and the unmodified starch were mixed in a fluid-bed process and the thus obtained powder was sprayed with a solution of HPMC in water. The homogeneously wetted granules thus obtained were dried, sieved together with microcrustalline cellulose, croscarmellose sodium, colloidal anhydrous silica and Mg stearate. The sieved powder was mixed and compressed to tablets. The coating suspension was prepared by first dissolving HPMC and propylene glycol in water and adding this solution to a homeginized mixture of water, talc, titanium dioxide and the dye. The coating suspension was sprayed onto the tablets in a coating apparatus at increased temperature.

| Tablet core of tablet: | | |
|---|---|---|
| ingredient | amount | % (w/w) versus tablet core |
| (+)-norcisapride-(L)-tartrate | 13.23 mg | 7.35 % (w/w) |
| lactose monohydrate 200 mesh(*1) | 107.73 mg | 59.85 % (w/w) |
| unmodified maize starch | 36.00 mg | 20.00 % (w/w) |
| HPMC 2910 15 mPa.s(*2) | 3.60 mg | 2.00 % (w/w) |
| microcrystalline cellulose | 12.60 mg | 7.00 % (w/w) |
| croscarmellose sodium | 5.40 mg | 3.00 % (w/w) |
| colloidal anhydrous silica | 0.54 mg | 0.30 % (w/w) |
| magnesium stearate | 0.90 mg | 0.50 % (w/w) |
| total weight of the tablet core : | 180.00 mg | |
| Film coating of tablet: | | |
| ingredient | amount | % (w/w) versus coating |
| HPMC 2910 15mPa.s | 4.00 mg | 55.95 % (w/w) |
| Propylene glycol | 1.00 mg | 13.99 % (w/w) |
| Titanium dioxide (E171) | 1.20 mg | 16.78 % (w/w) |

(*1) 200 mesh is an indication of the type of lactose monohydrate that is used.

(*2) HPMC means hydroxypropyl methylcellulose, the number "2910" refers to the type of hydroxypropyl methylcellulose that is used. The first two digits,"29", represent the approximate percentage of methoxylgroups and the third and fourth digit,"10" represents the approximate percentage of hydroxypropylgroups..

(continued)

| Film coating of tablet: | | |
|---|---|---|
| ingredient | amount | % (w/w) versus coating |
| Talc | 0.80 mg | 11.19 % (w/w) |
| Yellow Ferric Oxide (E172/C177492) | 0.15 mg | 2.10% (w/w) |
| total weight of coating : | 7.15 mg | |

Also indicated is the viscosity (15 mPa.s) of a 2% aqueous solution measured at 20 °C. This is an indication of the molecular weight of the HPMC that is used.

<u>C.2 Non-coated tablet.</u>

The ingredients were mixed in a planetary mixer and compressed in a tabletting apparatus.

| ingredient | amount | % (w/w) |
|---|---|---|
| Tablet C.2.1 | | |
| (+)-norcisapride | 10.387 mg | 5.8 |
| lactose monohydrate 200 mesh | 110.393 mg | 61.3 |
| unmodified maize starch | 36 mg | 20.0 |
| microcrystalline cellulose | 18 mg | 10.0 |
| polyvidone K90 | 3.6 mg | 2.0 |
| magnesium stearate | 0.9 mg | 0.5 |
| colloidal anhydrous silica | 0.54 mg | 0.3 |
| polysorbate 20 | 0.18 mg | 0.1 |
| Total weight of the tablet: | 180.00 mg | |
| Tablet C.2.2 | | |
| (+)-norcisapride-(L)-tartrate | 13.23 mg | 7.35 |
| MICROCELAC® | 157.23 mg | 87.35 |
| croscarmellose sodium | 7.2 mg | 4.00 |
| colloidal anhydrous silica | 0.54 mg | 0.3 |
| Mg stearate | 1.8 mg | 1.00 |

## Claims

**1.** Use of (+)-norcisapride for the manufacture of a medicament for treating 5-$HT_4$-mediated disorders.

**2.** Use according to claim 1 wherein the disorder is hampered or impaired gastrointestinal transit.

**3.** Use according to claim 1 wherein the disorder is hampered or impaired gastric emptying.

**4.** Use according to claim 1 wherein the disorder is gastro-oesophageal reflux

**5.** Use according to claim 1 wherein the disorder dyspepsia or gastroparesis.

**6.** Use (+)-norcisapride for the manufacture of a medicament for treating eating disorders.

**7.** Use according to claim 6 wherein the eating disorder is anorexia.

**8.** Use of (+)-norcisapride for the manufacture of a medicament suitable for accelerating intestinal cleansing with a laxative.

**9.** A use according to claim 8 wherein the laxative is an osmotic agent.

**10.** A use according to claim 8 wherein the laxative is a polyethylene glycol (PEG)-electrolyte solution.

**11.** Use of (+)-norcisapride for the manufacture of a medicament for treating 5-$HT_3$-mediated disorders.

**12.** Use according to claim 11 wherein the disorder is irritable bowel syndrome or diarrhea-predominant irritable bowel syndrome.

## Patentansprüche

**1.** Verwendung von (+)-Norcisaprid zur Herstellung eines Arzneimittels zur Behandlung von 5-$HT_4$-vermittelten Erkrankungen.

**2.** Verwendung nach Anspruch 1, wobei es sich bei der Erkrankung um gestörten oder behinderten Magen-Darm-Transport handelt.

**3.** Verwendung nach Anspruch 1, wobei es sich bei der Erkrankung um die gestörte oder behinderte Entleerung des Magens handelt.

**4.** Verwendung nach Anspruch 1, wobei es sich bei der Erkrankung um gastrointestinalen Reflux handelt.

**5.** Verwendung nach Anspruch 1, wobei es sich bei der Erkrankung um Dyspepsie oder Gastroparese handelt.

**6.** Verwendung von (+)-Norcisaprid zur Herstellung eines Arzneimittels für die Behandlung von Essstörungen.

**7.** Verwendung nach Anspruch 6, wobei es sich bei der Essstörung um Anorexie handelt.

**8.** Verwendung von (+)-Norcisaprid zur Herstellung eines Arzneimittels, das sich für die Beschleunigung der Darmabführung mit einem Laxativum eignet.

**9.** Verwendung nach Anspruch 8, wobei es sich bei dem Laxativum um ein Osmotikum handelt.

**10.** Verwendung nach Anspruch 8, wobei es sich bei dem Laxativum um eine Polyethylenglykol-(PEG)-Elektrolyt-Lösung handelt.

**11.** Verwendung von (+)-Norcisaprid zur Herstellung eines Arzneimittels zur Behandlung von 5-$HT_3$-vermittelten Erkrankungen.

**12.** Verwendung nach Anspruch 11, wobei es sich bei der Erkrankung um Colon irritabile bzw. um von Durchfallerscheinungen geprägtes Colon irritabile handelt.

## Revendications

**1.** Utilisation du (+)-norcisapride pour la fabrication d'un médicament destiné au traitement de troubles à médiation par la 5-$HT_4$.

**2.** Utilisation selon la revendication 1, dans laquelle le trouble est un transit gastro-intestinal entravé ou altéré.

**3.** Utilisation selon la revendication 1, dans laquelle le trouble est une vidange gastrique entravée ou altérée.

**4.** Utilisation selon la revendication 1, dans laquelle le trouble est un reflux gastro-oesophagien.

**5.** Utilisation selon la revendication 1, dans laquelle le trouble est la dyspepsie ou la gastroparésie.

**6.** Utilisation du (+)-norcisapride pour la fabrication d'un médicament destiné au traitement de troubles de l'alimentation.

7. Utilisation selon la revendication 6, dans laquelle le trouble de l'alimentation est l'anorexie.

8. Utilisation du (+)-norcisapride pour la fabrication d'un médicament approprié pour l'accélération du nettoyage intestinal à l'aide d'un laxatif.

9. Utilisation selon la revendication 8, dans laquelle le laxatif est un agent osmotique.

10. Utilisation selon la revendication 8, dans laquelle le laxatif est une solution électrolytique de polyéthylèneglycol (PEG).

11. Utilisation du (+)-norcisapride pour la fabrication d'un médicament destiné au traitement de troubles à médiation par la $5\text{-}HT_3$.

12. Utilisation selon la revendication 11, dans laquelle le trouble est le syndrome du côlon irritable ou le syndrome du côlon irritable à prédominance diarrhéique.